# EUROPÄISCHE PATENTSCHRIFT

(11) **EP 1 206 483 B1**
(45) Veröffentlichungstag und Bekanntmachung des Hinweises auf die Patenterteilung: **09.07.2003**
(21) Anmeldenummer: 00949438.6
(22) Anmeldetag: 02.08.2000
(51) Int. Cl.: C07K 1/36, B01D 29/01, B01D 61/18, B01L 9/06, B01L 3/00

(54) **AUTOMATISIERTE PROTEINREINIGUNG IM MULTIWELLFORMAT DURCH VAKUUMFILTRATION**
AUTOMATED PROTEIN PURIFICATION IN THE MULTIWELL FORMAT BY VACUUM FILTRATION
PURIFICATION AUTOMATISEE DE PROTEINES PAR FILTRATION SOUS VIDE AU MOYEN D'UNE PLAQUE DE MICROTITRATION A PUITS MULTIPLES

(30) Priorität: 06.08.1999 DE 19937187
(43) Veröffentlichungstag der Anmeldung: 22.05.2002
(73) Patentinhaber: QIAGEN GmbH, 40724 Hilden (DE)
(72) Erfinder: RIBBE, Joachim, D-40223 Düsseldorf (DE); SCHÄFER, Frank, 40627 Düsseldorf (DE); STEINERT, Kerstin, D-40764 Langenfeld (DE); LUBENOW, Helge, D-50859 Köln (DE)
(86) Internationale Anmeldenummer: EP0007478
(87) Internationale Veröffentlichungsnummer: WO01010886

(56) Entgegenhaltungen:
- EP-A- 0 249 932
- EP-A- 0 339 769
- DE-A- 19 834 584

## Beschreibung

Die vorliegende Erfindung betrifft ein Verfahren sowie ein Reagenzienkit zur Gewinnung von Zellinhaltsstoffe enthaltenden klaren Lösungen aus biologischen Proben im Hochdurchsatzverfahren, sowie zur Gewinnung von Zellinhaltsstoffen aus den erhaltenen klaren Lösungen. Weiterhin betrifft die Erfindung die Verwendung einer automatisierbaren Vorrichtung zur Gewinnung von klaren proteinhaltigen Lösungen.

Auf dem Gebiet der Biochemie bzw. Molekularbiologie besteht, insbesondere in Screenings, oftmals die Notwendigkeit, eine Vielzahl biologischer Proben näher zu untersuchen, um beispielsweise gewünschte Klone zu identifizieren oder die Gegenwart bzw. den Gehalt von Zellinhaltsstoffen in biologischen Proben zu bestimmen. Zur Verringerung des Zeitbedarfs und somit der Kosten sowie der Fehlermöglichkeiten wurden in der Vergangenheit Verfahren bzw. Systeme entwickelt, die den Verfahrensablauf vereinfachen. Ein Ansatz zur Verringerung des Arbeitsaufwands bestand in der Bereitstellung von Systemen, die eine parallele Bearbeitung mehrerer Proben ermöglichen, wie etwa Mikrotiterplatten, dafür geeigneten Mehrfachpipetten oder Rotoreinsätzen, welche Mikrotiterplatten aufnehmen können. Ein weiterer Ansatz zielt darauf ab, einzelne Verfahrensschritte weitgehend zu automatisieren, wie durch den Einsatz geeigneter Automaten.

Pelleisen et al. (Biotechniques 20, 616-620 (1996), beschreiben ein Verfahren zur Reinigung von rekombinanten Maltose-Binding-Protein (MBP)-Fusionsproteinen im 96-Well-Format. Das Verfahren geht aus von klaren Zellysaten, die auf herkömmliche Weise hergestellt werden und in Wells einer 96-Well-Platte aufgetragen werden, Bindung der im Überstand enthaltenen MBP-Fusionsproteine an Maltose-beschichtete Oberflächen und Elution der MBP-Fusionsproteine durch Zugabe von Maltoseüberschuß oder denaturierenden Reagenzien. Nachteilig bei diesem Verfahren ist, daß es nicht automatisiert und aufgrund der Herstellung von klaren Zellysaten durch Zentrifugation auch nicht automatisierbar ist. Das Verfahren ist nur unter nativen, jedoch nicht unter denaturierenden Bedingungen verwendbar und führt zu einer nur geringen Ausbeute (ca. 2 µg pro Well).

Bell et al. (http://www.apbiotech.com/publications/LSN-1-5/gst-micro.htm, 1999) beschreiben ein Verfahren zur parallelen Reinigung von bis zu 24 rekombinanten Glutathion-S-Transferase-(GST)-Fusionsproteinen. Klare Zellysate werden durch Zentrifugation hergestellt und auf separate Mikro-Chromatographiesäulen aufgetragen. Die nachfolgenden Bindungs- und Waschschritte werden durch Zentrifugation oder durch Anlegen eines Unterdrucks ausgeführt. Elution erfolgt durch Zugabe von reduziertem Glutathion in einzelne Mikroreaktionsgefäße. Nachteilig bei diesem Verfahren ist, daß es nicht automatisiert ist und aufgrund der Herstellung klarer Zellysate durch Zentrifugation auch nicht automatisierbar ist. Das Verfahren ist nur unter nativen Bedingungen durchführbar und erlaubt eine parallele Verarbeitung von lediglich 24 Proben. Zudem besteht durch die Verwendung von einzelnen Reaktionsgefäßen eine hohe Verwechslungsgefahr.

Sheer und Pitt (High Throughput Sample Preparation of Proteins and Peptides for Structural Characterization, Poster Nr. 446-T, 13-15 Juli 1997, 11th Symposium of the Protein Society, Boston, MA) beschreiben ein Verfahren zum Beladen der 96 Wells einer 96-Well-Mikrotiterplatte mittels eines "Multiscreen Column Loader" mit trockenem, pulverförmigen Chromatographiematerial. Nach Schwellen und Äquilibrieren des Materials können 96 Ansätze parallel chromatographisch bearbeitet werden, wobei alle Schritte durch Zentrifugation durchgeführt werden. Dieses Verfahren betrifft nur die chromatographische Reinigung, nicht jedoch die Probenvorbereitung.

EP-A-0 339 769 schreibt ein Verfahren zur Reinigung von Proben zu diagnostischen Zwecken mit Hilfe von Multifilter-Streifen in einer Multikammer-Filtration. Es werden zwei wesentlich analytische Verfahren mit mikroporigen Membranen verwendet. Hierbei benutzt das erste Verfahren die mikroporige Membran zur Ansammlung von kleinen Partikeln zur Analyse, während im zweiten Verfahren z. B. eine Bindung der einzelsträngigen RNA oder DNA an die Nitrozellulosemembran für Nukleinsäurehybridisierungen durchgeführt wird.

Die vorstehend genannten Dokumente beschreiben die Reinigung von Proteinen in parallelen Ansätzen, sind aber sämtlich nicht automatisiert und in der beschriebenen Form nicht automatisierbar. Als Ausgangsmaterial werden jeweils klare Zellysate eingesetzt, welche auf herkömmliche Weise, d.h. durch Zentrifugation der einzelnen Proben und Abnahme der Überstände hergestellt worden sind. Dies impliziert die Erfordernis zahlreicher Handlingschritte und in Folge dessen einen hohen Zeitbedarf und die Verwechslungsgefahr von Proben.

DE 42 30 089 beschreibt einen Laborroboter zur vollautomatischen Aufarbeitung von bis zu maximal 30 synthetischen Peptiden aus der Festphasensynthese. Eine Aufarbeitung von biologischem Material wie etwa Zellen oder Zellextrakten ist nicht offenbart.

EP-A-0 376 080 beschreibt ein Verfahren zur Extraktion und Reinigung von DNA ohne Zentrifugationsschritte. Eine parallele Aufarbeitung mehrerer Proben, insbesondere im Hochdurchsatzverfahren, wird nicht offenbart.

EP-A-0 249 932 beschreibt ein automatisiertes Verfahren zur Reinigung physiologisch aktiver Substanzen im Prozessmaßstab. Das Verfahren beruht auf einer Makrofiltrationsabtrennung von Zellen oder Zellbestandteilen, einer Ultrafiltration zur Abtrennung von Substanzen mit einem niedrigeren Molekulargewicht als die zu isolierende Substanz, sowie einem Affinitätschromatographieschritt. Eine parallele Reinigung von Substanzen, insbesondere im Hochdurchsatzverfahren ist nicht offenbart.

Die vorstehenden europäischen Patentanmeldungen beschreiben automatisierbare Verfahren zur Reinigung von DNA bzw. Proteinen aus Zellen bzw. Zellsuspensionen. Sie offenbaren jedoch ausschließlich Verfahren zur Reinigung dieser Substanzen aus einer einzelnen Probe und nicht im Hochdurchsatzverfahren, und sie berücksichtigen dementsprechend auch nicht die bei Hochdurchsatzanwendungen auftretenden besonderen Gegebenheiten.

Bedingt durch den geringen räumlichen Abstand der separaten Proben besteht ein erhebliches Problem bei Hochdurchsatzanwendungen in einer Kreuzkontamination durch Lösungen aus benachbarten Wells bzw. Kammern. Diese Gefahr besteht insbesondere bei Filtrationsschritten durch eine Muttikammer-Filtrationseinheit, vor allem wenn die zu filtrierende Lösung zu starker Schaumbildung neigt. Insbesondere relevant ist dieses Problem bei der Herstellung von klaren Lysaten aus Zellkulturen. Aus diesem Grund gehen bekannte Parallelverfahren bereits von klaren Zellysaten aus, die auf herkömmliche Weise, d.h. durch Zentrifugation erhalten wurden, vergl. den vorstehend genannten Stand der Technik.

Die der Erfindung zugrundeliegende Aufgabe bestand somit darin, ein einfaches und schnelles Verfahren zur parallelen Gewinnung von klaren Lösungen aus biologischen Proben, insbesondere aus Zellen bzw. Zellsuspensionen oder -rohlysaten bereitzustellen.

Ein Gegenstand der Erfindung ist somit ein Verfahren zur Gewinnung von Zellinhaltsstoffe enthaltenden klaren Lösungen aus biologischen Proben im Hochdurchsatzverfahren, umfassend die Schritte
(a) Bereitstellen von proteinhaltigen Lösungen, die unlösliche Bestandteile enthalten, in separaten Kammern einer Multikammer-Filtrationseinheit,
(b) Entfernen von unlöslichen Bestandteilen durch Filtrieren der Lösungen durch die Multikammer-Filtrationseinheit unter Anlegen einer Druckkdifferenz, wobei durch Zugabe eines Entschaumers zu den Zelllysaten und/oder durch Verwendung einer Transfereinheit zwischen Auslassseite der Multikammer-Filtrationseinheit und Einlassseite der Auffangbehälter eine Kreuzkontamination benachbarter Kammern verhindert wird, und
(c) Auffangen der einzelnen Filtrate jeweils separat in Auffangbehältern.

Unter einer biologischen Probe wird im Rahmen dieser Anmeldung eine Probe, die biologisches Material enthält, verstanden. Das biologische Material stammt beispielsweise aus Geweben jeder Art, Knochenmark, humanen und tierischen Körperflüssigkeiten wie Blut, Serum, Plasma, Urin, Sperma, Cerebrospinalflüssigkeit, Sputum und Abstrichen, Pflanzen, Pflanzenteilen und -extrakten, z.B. Säften, Pilzen, Mikroorganismen, wie Bakterien oder Viren, pro- oder/und eukaryontischen Zellen oder Zellkulturen, fossilen oder mumifizierten Proben, Bodenproben, Klärschlamm, Abwässern und Lebensmitteln. Insbesondere kann die biologische Probe rekombinantes Material, beispielsweise mit rekombinanten Techniken veränderte eukaryontische oder/und prokaryontische Zellen umfassen.

Die proteinhaltige Lösung enthält Peptide oder/und Polypeptide aus dem biologischen Material in löslicher Form. Beispielsweise sind diese löslichen Peptide und/oder Polypeptide sekretierte Substanzen, die in der biologischen Probe vorhanden sind oder/und von Zellen in der biologischen Probe sekretiert werden. Alternativ können sie Peptide oder/und Polypeptide sein, die innerhalb von Zellen vorkommen oder rekombinant exprimiert werden, und durch ein Aufbrechen von Zellen freigesetzt worden sind. In einer besonderen Ausführungsform sind die proteinhaltigen Ausgangslösungen (d.h. Lösungen, die unlösliche Bestandteile enthalten) Zellrohlysate, wobei die Lyse unter Protein-solubilisierenden Bedingungen durchgeführt wird. Weiterhin können die proteinhaltigen Ausgangslösungen auch Fraktionen von Zellrohlysaten darstellen. Ein Beispiel für eine Gewinnung einer Lysatfraktion ist beispielweise die Durchführung der Lyse unter Proteinpräzipitierenden Bedingungen und Abtrennen der Nukleinsäuren, und erneutes Aufnehmen des Rückstands unter Protein-solubilisierenden Bedingungen.

Die Begriffe "klare Lösung" und "Filtrat" werden hier in synonym verwendet und bezeichnen eine Lösung, die im wesentlichen frei von unlöslichen Bestandteilen, wie z.B. Zelltrümmern, ist.

Die Lösungen werden im allgemeinen verschiedenen Ursprungs sein, es sind jedoch auch Anwendungen denkbar, wobei Aliquots aus einer identischen Probe parallel bearbeitet werden.

Unter den Begriff "Hochdurchsatz" wird hierin eine parallele Verarbeitung von mindestens 24, bevorzugt mindestens 48 und am meisten bevorzugt 96 Proben und mehr, beispielsweise 384 Proben, verstanden.

Gemäß einer Ausführungsform verhindert man die Kreuzkontamination durch Verringerung von Schaumbildung. Eine solche Schaumbildung tritt bei Filtration einer Proteine enthaltenden Lösung am Auslaß der einzelnen Kammern einer Multikammer-Filtrationseinheit auf. Diese sich bildende Schaumkrone kann sich mit dem Schaum an benachbarten Kammerauslässen verbinden und zu Kreuzkontaminationen führen. Schaumbildung wird beobachtet bei der Filtration von Lysaten einer Proteinkonzentration von ≥ 0,1 mg pro ml Lysat. Ebenso wird Schaumbildung beobachtet bei der Filtration von (komplexen) Kulturmedien, die Proteine enthalten. Solche Kulturmedien sind dem Experten bekannt und enthalten z.B. Extrakte von Hefezellen (z.B. LB-, TB-, dYT-, YP-Medien), angedaute Extrakte tierischer und pflanzlicher Gewebe (z.B. Tryptone, Peptone, Soja-Pepton), Hydrolysate von z.B. Casein, Milchalbumin oder Gelatin, Blutserumbestandteile, oder sind proteinangereicherte serumfreie Medien (z.B. CHO-S-SFMII). Zu starker Schaumbildung kommt es bei Proteingehalten von ≥ 0,01 mg pro ml Medium.

In einer bevorzugten Ausführungsform der Erfindung wird die Schaumbildung durch Zugabe eines Entschäumers zu den proteinhaltigen Lösungen, insbesondere durch Überschichten der proteinhaltigen Lösungen mit Entschäumer, verringert. Geeignete Entschäumer sind beispielsweise primäre, sekundäre oder tertiäre Alkohole, insbesondere C1-C6 Alkohole, Entschäumer auf Silikonölbasis oder Emulsionen davon, wie beispielsweise Antifoam A (SIGMA), Wacker Silikon-Antischaumemulsion SRE (Antischaumreagenz DX, Wacker) oder Dow Corning Antifoam MSA Compound (Dow Corning) nichtsilikonische organische Entschäumer oder Emulsionen davon, z.B. Antifoam 204 (SIGMA), Entschäumer auf vegetabilischer Basis, insbesondere alkoxylierte Fettsäureester, z.B. Struktol J673 (Schill und Sailacher), langkettige Alkane (C6 und länger), Mineralöle, Polyglykole wie Polypropylenglykole und Polyethylenglykole, sowie Gemische davon, beispielsweise Gemische, die silikonische und nichtsilikonische Entschäumer enthalten, z.B. Antifoam 289 (SIGMA). Bevorzugt ist der Entschäumer ein Alkohol, insbesondere Ethanol oder Isopropanol.

Die Konzentration an Entschäumer variiert in Abhängigkeit des Typs der Entschäumersubstanz, beispielsweise werden Alkohole, insbesondere Ethanol, in konzentrierter Form verwendet, während etwa Entschäumer auf Silikonölbasis in einer Konzentration um 0,1 % eingesetzt werden.

Wenn die Zugabe des Entschäumers gemäß der bevorzugten Ausführungsform durch Überschichten erfolgt, richtet sich das notwendige Volumen an Entschäumer nach dem Durchmesser der Kammern bzw. der Flüssigkeitssäule an Entschäumer, d.h. es ist größenteils unabhängig vom Volumen der proteinhaltigen Lösungen. Weiterhin ist das Volumen auch vom Type des Entschäumers abhängig. Bei Verwendung von Ethanol als Entschäumer wurde festgestellt, daß eine Säulenhöhe im Bereich von 0,05 cm bis 1 cm, bevorzugt 0,1 cm bis 0,3 cm und insbesondere etwa 0,2 cm zu guten Ergebnissen führt (bezogen auf einen Durchmesser der Kammern von 0,8 cm, bei Änderung des Durchmessers der Kammern können Anpassungen in Bezug auf die Säulenhöhe angebracht sein).

Gemäß einer anderen Ausführungsform der Erfindung verhindert man die Kreuzkontamination durch Verwendung einer Multikammer-Transfereinheit zwischen der Auslaßseite der Multikammer-Filtrationseinheit und der Einlaßseite der Auffangbehälter. Unter "Multikammer-Transfereinheit" wird eine Einheit verstanden mit separaten Durchgängen, deren Einlaßöffnungen den Auslaßöffnungen der einzelnen Kammern der Multikammer-Filtrationseinheit korrespondieren und deren Auslaßöffnungen den Einlaßöffnungen der Auffangbehälter korrespondieren. Einlaßseitig schließt die Transfereinheit im wesentlichen dichtend zu den Auslaßöffnungen der Multikammer-Filtrationseinheit ab um eine Kreuzkontamination durch Schaumbildung zu verhindern. Auslaßseitig kann ein nicht-dichtender Sitz zu den Einlaßöffnungen der Auffangbehälter vorgesehen sein, um das Anlegen einer Druckdifferenz zwischen Einlaß- und Auslaßseite der Multikammer-Filtrationseinheit zu ermöglichen. Alternativ kann auslaßseitig der Multikammer-Filtrationseinheit ein dichtender Sitz zur Einlaßseite der Auffangbehälter vorgesehen sein, etwa um das Anlegen einer Druckdifferenz zwischen Einlaßseite der Multikammer-Filtrationseinheit und einer möglichen Auslaßseite der Auffangbehälter zu ermöglichen. Wenn die Auffangbehälter eine Chromatographhiematrix enthalten, können so Filtration der Ausgangslösungen und eine Weiterbarbeitung, wie z.B. Bindung von zu reinigenden Zellinhaltsstoffen an die Matrix in einem Vakuumschritt durchgeführt werden (siehe nachstehend). Die Transfereinheit ist beispielsweise eine Dichtungsmatte, ein mit durchgehenden Bohrungen versehener Aluminium-, Glas- oder Kunststoff-Block, oder eine Anordnung entsprechender Röhren oder Schläuche.

In einer besonderen Ausführungsform geht das erfindungsgemäße Verfahren aus von Zell-Rohlysaten, die in separaten Kammern einer Multikammer-Filtrationseinheit bereitgestellt werden. Die Zellysate können vorab zubereitet worden sein durch Mischen von Zellen oder Zellsuspensionen mit Lysereagenzien, oder die Lyse kann direkt in den einzelnen Kammern der Multikammer-Filtrationseinheit durchgeführt werden durch Zugabe von Zellen und Lysesubstanzen und gegebenenfalls Inkubation. Die Lyse erfolgt dabei unter Protein-solubilisierenden Bedingungen oder beinhaltet nach Abtrennung einer oder mehrerer Fraktionen einen Solubilisierungsschritt für Proteine.

Die Abtrennung der proteinhaltigen Lösungen von den unlöslichen Bestandteilen erfolgt durch Anlegen einer Druckdifferenz zwischen Einlaßseite- und Auslaßseite der Multikammer-Filtrationseinheit, wobei auslaßseitig ein geringerer Druck vorliegt. Die Druckdifferenz kann erzeugt werden durch Anlegen eines einlaßseitigen Überdrucks oder bevorzugt eines auslaßseitigen Vakuums.

Der in den separaten Kammern der Multikammer-Filtrationseinheit vorhandene Filter ist bevorzugt ein Filter dessen Porengröße in Fließrichtung der Filtrate durch den Filter abnimmt. Ein derartiger Filter ist in EP 0 616 638 B1 beschrieben, worauf hiermit für weitere Details verwiesen wird. Er vereint die Vorteile eines grobporigen Filters (geringe Verstopfung der Filterporen) mit denjenigen eines engporigen Filters (gute Rückhaltung feiner unlöslicher Bestandteile). Ein herkömmlicher Filter mit einheitlicher Porengröße ist jedoch ebenfalls geeignet.

Nach dem Filtrationsschritt werden die Filtrate jeweils getrennt in Auffangbehältern, zweckmäßig in den separaten Kammern einer Multikammer-Einheit aufgenommen. Insbesondere wenn eine Weiterverarbeitung der klaren Lösungen vorgesehen ist (siehe nachstehend), wird diese Multikammer-Einheit im allgemeinen eine weitere Filtrations- oder eine Chromatographie-Einheit sein.

In einer besonderen Ausführungsform umfaßt das erfindungsgemäße Verfahren weiterhin das Gewinnen von Zellinhaltsstoffen aus derart gewonnen Filtraten. Hierzu umfaßt das Verfahren beispielsweise einen oder mehrere weitere Filtrationsschritte, beispielsweise eine Ultrafiltration, wodurch eine Abtrennung von Substanzen erreicht wird, die ein geringeres Molekulargewicht als die gesuchten Zellinhaltsstoffe aufweisen, sowie eine Einengung des Probenvolumens.

Zur Gewinnung von Zellinhaltsstoffen umfaßt das Verfahren bevorzugt mindestens einen chromatographischen Trennschritt oder/und Präzipitationsschritt. Ein Präzipitationsschritt umfaßt beispielsweise die Zugabe von Ethanol in ausreichender Konzentration um Nukleinsäuren zu fällen oder von Ether zur Fällung von Peptiden. Ein chromatographischer Trennschritt beinhaltet die Bindung der gewünschten Zellinhaltsstoffe an ein geeignetes Chromatographiematerial, das Entfernen der Überstände und anschließend die Elution der Inhaltsstoffe.

Das Chromatographiematerial unterliegt keinen besonderen Beschränkungen und wird in Abhängigkeit von den abzutrennenden Zellinhaltsstoffen ausgewählt. Beispielsweise kann das Chromatographiematerial eine lonenaustauschmatrix sein.

Geeignete Matrixmaterialien umfassen z.B. Agarose, Silika, Nitrocellulose, Cellulose, Acrylamid, Latex, Polystyrol, Polyacrylat, Polymethacrylat, Polyethylen-Polymere wie Polyvinylalkohol, Glaspartikel, Silikate wie z.B. Calcium-, Magnesium- und Aluminium-Silikate, Metalloxide wie z.B. Titanoxide, Apatite, und Kombinationen daraus. Bevorzugte Matrixmaterialen sind Silikagel oder/und Agarose.

In einer nochmals bevorzugten Ausführungsform umfaßt das erfindungsgemäße Verfahren zur Gewinnung von Zellinhaltsstoffen einen chromatographischen Trennschritt über eine Affinitätsmatrix. Derartige Affinitätsmatrices sind dem Fachmann bekannt und umfassen beispielsweise die in der nachfolgenden Liste angegebenen Ligandensysteme (gekoppelt an ein Matrixmaterial, siehe oben).
- Affinitätsligand: damit zu isolierendes Molekül
- Antigen: bestimmter Antikörper
- Antikörper: bestimmtes Antigen
- Antikörper: bestimmter Antikörper
- Antikörper (z.B. anti-Mensch IgG, anti-Maus IgG): bestimmte Klasse von Antikörpern wie z.B. IgG Moleküle aus Mensch o. Maus
- Protein A oder Protein G: bestimmte Klassen von Antikörpern
- Streptavidin oder Streptavidin- markierte Fusionsproteine: Biotin, Avidin, Biotin- oder Avidin- markierte Fusionsproteine
- Glutathion: Gluthathion-S-Transferase (GST) oder GST-Fusionsproteine
- Cellulose: Cellulose-bindende Domäne (CBD) oder CBD-Fusionsproteine
- Calmodulin: Calmodulin-bindendes Protein (CBP) oder CBP-Fusionsproteine
- Amylose: Maltose-bindendes Proteine (MBP) oder MBP-Fusionsproteine
- lonentauschergruppen: Biomoleküle
- Ligand für hydrophobe Interaktionen: Biomoleküle
- oligo dT: polyA-Bereiche, z.B. von mRNA-Molekülen
- Nukleinsäuren: hybridisierende Nukleinsäure
- Nukleinsäuren, definierte Sequenz: spezifisch Nukleinsäure-bindende Biomoleküle, z.B. Proteine
- Protein: Interaktionspartner (Protein, Nukleinsäuren allgem., Nukleinsäuren bestimmter Sequenz, kleine Biomoleküle)
- kleine Biomoleküle: Proteine, Nukleinsäuren
- Zell-bindende Liganden, z.B. Polypeptid: Zellen über die Bindung von Zelloberflächenmolekülen
- Phagen-bindende Liganden, z.B. Polypeptid: Phagen über die Bindung von Molekülen auf der Phagenoberfläche
- Antikörper: Zellen oder Phagen über die Bindung von Oberflächenmolekülen

Ein weiteres Beispiel für Affinitätsmatrices ist eine Metallchelat-Affinitätsmatrix zur Reinigung von Proteinen bzw. Peptiden. Unter Verwendung einer Ni-NTA-Matrix (erhältlich von Qiagen) wurden hervorragende Ergebnisse bei der Reinigung von 6xHis-markierten Proteinen erzielt (siehe Beispiele).

Die Form des Matrixmaterials ist nicht besonders eingeschränkt, es können Materialien in Suspensions- oder Membranform eingesetzt werden. Beispiele dafür sind, jeweils funktionalisiert, Membranen, (monodisperse) Latexbeads, continuousbed-Material, Polymerchromatographiemedien, Silikapartikeletc. Unter "funktionalisiert" wird die Gegenwart einer Gruppe verstanden, die zur Ausbildung einer affinen Wechselwirkung mit den gesuchten Zellinhaltsstoffen fähig ist. Ein spezifisches Beispiel für ein Chromatographiemedium vom Suspensionstyp ist etwa Ni-NTA-Superflow (erhältlich von Qiagen).

Das erfindungsgemäße Verfahren zur Gewinnung von Zellinhaltsstoffen beinhaltet zweckmäßig einen oder mehrere Waschschritte. Die Abtrennung von Flüssigkeiten, d.h. des Überstands nach Präzipitation bzw. Bindung der gewünschten Zellinhaltsstoffe an ein Chromatographiematerial, sowie der Waschflüssigkeit wird erfindungsgemäß bevorzugt durch Anlegen einer Druckdifferenz, insbesondere eines auslaßseitigen Unterdrucks durchgeführt. Soweit erforderlich werden bei diesen weiteren Filtrations- oder/und Chromatographieschritten geeignete Maßnahmen getroffen, um eine Kontaminaton durch Flüssigkeiten in benachbarten Kammern zu verhindern, beispielsweise durch Zugabe eines Entschäumers oder durch Verwendung einer Transfereinheit.

In einer besonderen Ausführungsform werden die zu verwerfenden Flüssigkeiten (Waschflüssigkeiten etc.) unter Verwendung einer Drainageeinheit entfernt. Diese Drainageeinheit entspricht einlaßseitig der vorstehend beschriebenen Multikammer-Transfereinheit, d.h. die Überstände werden durch separate Durchgänge von der Auslaßseite einer Multikammer-Filtrationseinheit weg-geführt. Auslaßseitig werden diese Flüssigkeiten durch die Drainageeinheit einem Abfall-Sammelgefäß zugeführt.

Nach Durchführung des erfindungsgemäßen Verfahrens zur Gewinnung bzw. Reinigung/Isolation von Zellinhaltsstoffen werden die Lösungen, welche die Zellinhaltsstoffe enthalten, separat in Auffangbehältern, insbesondere in den separaten Kammern einer Multikammer-Einheit, aufgenommen.

Das erfindungsgemäße Verfahren kann weiterhin Schritte zur Analyse oder/und Konservierung der Zellinhaltsstoffe umfassen. Analyseschritte umfassen im allgemeinen die Entnahme z.B. eines Aliquots und dessen Zuführung einer Elektrophorese-Einheit, z.B. SDS-PAGE oder/und einer Spektroskopie-Einheit, z.B. einem Massenspektrometer oder einem Assay. Ein spezifisches Beispiel für einen Konservierungsschritt ist Gefriertrocknung.

Das erfindungsgemäße Verfahren ist zur Gewinnung einer Vielzahl von Zellinhaltsstoffen geeignet, insbesondere zur Gewinnung von Peptiden, Polypeptiden, Nukleinsäuren oder/und Metaboliten. Voraussetzung dafür ist ein geeignetes Protokoll zur Abtrennung der Zellinhaltsstoffe von anderen Substanzen unter Verwendung eines oder mehrerer Filtrations-, Präzipitations- und/oder chromatographischer Trennschritte. Bevorzugt wird das Verfahren zur Gewinnung von Peptiden oder Polypeptiden eingesetzt, wobei Polypeptide im allgemenen mindestens 50 Aminosäuren enthalten.

Zu den Polypeptiden gehören beispielsweise Proteine, Glykoproteine und Lipoproteine, die gegebenenfalls aus mehreren Untereinheiten zusammengesetzt sind oder/und prosthetische Gruppen enthalten, wie Antikörper oder Enzyme. Darüber hinaus kann durch geeignete Kombination von Verfahrensschritten eine Gewinnung mehrerer Zellinhaltsstoffe aus demselben Lysat vorgesehen werden. Beispielsweise können in einem Komplex von Verfahrensschritten Peptide oder Polypeptide durch Bindung an eine Affinitätsmatrix isoliert werden und in einem weiteren Komplex Nukleinsäuren, z.B. für diese Peptide oder Polypeptide kodierende Plasmid-DNA, gewonnen werden.

Das erfindungsgemäße Verfahren ist insbesondere vorgesehen zur raschen parallelen Aufarbeitung von Proben im Labormaßstab, d.h. für ein Fassungsvermögen von bis zu 50 ml, bevorzugt bis 20 ml und stärker bevorzugt von 0,1 bis 2 ml jeder Kammer der Multikammer-Filtrationseinheit, in der die proteinhaltigen Ausgangslösungen bereitgestellt werden.

Weitere Vorteile des erfindungsgemäßen Verfahrens neben der Vermeidung von Kreuzkontaminationen in einem Hochdurchsatzverfahren bestehen insbesondere in
- hoher Automatisierbarkeit der parallelen Herstellung klarer Zellysate,
- Vermeidung von manuellen Transferschritten oder/und Pipettierschritten,
- hoher Automatisierbarkeit der Gewinnung von Zellinhaltsstoffen aus Zell-Rohlysaten,
- guten Ausbeuten sowie hoher Reinheit der gewonnenen Zellinhaltsstoffe,
- prinzipieller Anwendbarkeit des Verfahrens sowohl unter nativen als auch unter denaturierenden Bedingungen,
- Verringerung der Fehlerquellen durch Automatisierung des Verfahrens und Verwendung von Multikammer-Einheiten,
- Schnelligkeit des Verfahrens,
- Flexibilität des Systems.

Das erfindungsgemäße Verfahren ist geeignet zur Gewinnung von klaren proteinhaltigen Lösungen bzw. zur Gewinnung von Zellinhaltsstoffen aus einer biologischen Probe, beispielseise Prokaryonten, wie z.B. E.coli, Eukaryonten wie z.B. Saccharomyces cerevisiae, pflanzlichen, tierischen und humanen Gewebe- und Kulturzellen, Insektenzellen, Pilzen, Archaea etc., sowie beispielsweise für automatisierte Phage-Display-Assays.

Praktische Anwendungsmöglichkeiten des erfindungsgemäßen Hochdurchsatzverfahrens (HT, High Throughput) liegen beispielsweise in:

### Functional Genomics:

- HT-Charakterisierung von Genen/offener Leserahmen auf Proteinebene im Rahmen von Genomsequenzierungsprojekten (z.B. Hefegenomprojekt, Human Genome Project)

### Functional Proteomics:

- HT-Reinigung rekombinanter Proteine und von Protein- und Peptidkomplexen
- HT-Reinigung rekombinanter Proteine und von Protein- und Peptidkomplexen als Probenvorbereitung für die Immobilisierung an Oberflächen (z.B. Mikrotiterplatten, Mikroreaktoren, Proteinchips [microarrays])
- HT-Reinigung rekombinanter Proteine als Probenvorbereitung für Wirkstoff-Screening-Programme
- HT-Reinigung rekombinanter Proteine und von Protein- und Peptidkomplexen als Probenvorbereitung für Interaktionsstudien, z.B. im Rahmen von Cell Maß Proteomics-Projekten
- Isolierung von Peptiden, Protienen, Peptid- oder Proteinkomplexen und anderer Biomoleküle (z.B. Nukleinsäuren, Kohlehydrate, Lipide) über die Wechselwirkung mit immobilisierten, z.B. 6xHis-markierten Proteinen, z.B. im Rahmen von Cell Maß Proteomics-Projekten, Wirkstoff-Screening-Programmen
- Isolierung von ganzen Organismen (Zellen, Phagen), die auf ihrer Oberfläche bestimmte Biomoleküle (z.B. Proteine) exprimieren, über deren spezifische Wechselwirkung mit immobilisierten Interaktionspartnern, z.B. an Ni-NTA-Matrix immobilisierte 6xHis-markierte Proteine (Affinity enrichment, expression cloning, panning).
- Genexpressionsanalysen auf Proteinebene (Protein Expression Profiling)

Ein weiterer Gegenstand der vorliegenden Erfindung ist die Verwendung einer Vorrichtung, umfassend
- Mittel zur Halterung einer Multikammer-Einheit in einer ersten Position,
- Mittel zur Halterung einer Multikammer-Einheit in einer zweiten Position,
- Mittel zum Anlegen einer Druckdifferenz zwischen der Einlaßseite und der Auslaßseite mindestens der Multikammer-Einheit in der ersten Position und
- Mittel zur Zugabe von Reagenzien zu einzelnen Kammern mindestens der Multikammer-Einheit in der ersten Position,
zur Gewinnung von klaren proteinhaltigen Lösungen aus biologischen Proben.

In der ersten Position wird eine Multikammer-Filtrationseinheit zur Aufnahme von proteinhaltigen Lösungen, die unlösliche Bestandteile enthalten, bereitgestellt. In der zweiten Position wird eine Multikammer-Auffangeinheit zum Auffangen der klaren proteinhaltigen Lösungen bereitgestellt. Insbesondere, wenn eine anschließende Gewinnung von Peptiden oder/und Polypeptiden aus den proteinhaltigen Lösungen vorgesehen ist, ist die Multikammer-Einheit in der zweiten Position im allgemeinen eine weitere Multikammer-Filtrationseinheit oder eine Multikammer-Chromatographie-Einheit.

Die Mittel zum Anlegen einer Druckdifferenz zwischen der Einlaßseite und der Auslaßseite, mindestens der Multikammer-Einheit in der ersten Position sind eine Vorrichtung zur Erzeugung eines einlaßseitigen Überdrucks oder/und eines auslaßseitigen Unterdrucks. Bevorzugt sind diese Mittel eine auslaßseitige Vakuumvorrichtung. Die Multikammereinheit in der ersten Position schließt die Einlaßseite von der Auslaßseite im wesentlichen dichtend ab, um die Erzeugung einer Druckdifferenz zu ermöglichen. Die Druckdifferenz kann zwischen Einlaßseite und Auslaßseite der Multikammereinheit in der ersten Position oder zwischen Einlaßseite der Multikammereinheit in der ersten Position und der Auslaßseite der Mulitkammer-Einheit in der zweiten Position erzeugt werden. Gegebenenfalls umfaßt die Vorrichtung weiterhin Mittel zum Anlegen einer Druckdifferenz zwischen Einlaßseite und Auslaßseite der Multikammer-Einheit in der zweiten Position.

Die Mittel zur Zugabe von Reagenzien umfassen insbesondere Pipettiermittel, bevorzugt Mehrfach-Pipettiermittel zur Zugabe von beispielsweise Lyselösungen, Waschpuffern etc. zu den einzelnen Kammern.

Die Multikammereinheiten in der ersten und zweiten Position sind im allgemeinen derart ausgerichtet, daß die Auslässe der Kammern einer Einheit in der ersten Position mit den Einlässen der Kammern einer Einheit in der zweiten Position fluchten. Dadurch wird ein einfacher Transfer von Lösungen aus den Kammern der Einheit in der ersten Position nach der Passage durch das Filtriermittel zu den Kammern der Einheit in der zweiten Position gewährleistet.

Bei Verwendung der Vorrichtung zur Gewinnung von Peptiden oder/und Polypeptiden wird in der ersten Position eine Multikammer-Filtrationseinheit und in der zweiten Position eine Multikammer-Auffangeinheit, bevorzugt eine weitere Filtrationseinheit oder Chromatographie-Einheit bereitgestellt. Nach Filtration der Lysate durch die Multikammer-Filtrationseinheit liegen die klaren Lösungen in den Kammern der zweiten Multikammer-Filtrationseinheit bzw. -Chromatographieeinheit vor und sind dort weiteren Manipulationen zugänglich. Für einen weiteren Filtrations- oder Chromatographie-Schritt sind, zumindest bei der bestimmten Ausführungsformen, zusätzliche Vakuumpumpen an der Auslaßseite der Multikammer-Einheit in der zweiten Position erforderlich. Durch Entnahme der Multikammer-Einheit in der ersten Position und Verschieben der Multikammer-Einheit aus der zweiten in die erste Position entfällt dieses Erfordernis. In einer besonderen Ausführungsform umfaßt die Vorrichtung somit
- Mittel zum Entfernen einer Multikammer-Einheit aus der ersten Position,
- Mittel zum Transferieren einer Multikammer-Einheit von der zweiten in die erste Position und
- Mittel zum Transferieren einer Multikammer-Einheit in die zweite Position.

Die zusätzliche, in die zweite Position eingebrachte dritte Multikammer-Einheit ist, je nach vorgesehener Verfahrensführung, eine Multikammer-Filtrationseinheit, -Chromatographieeinheit oder -Auffangeinheit.

Die Vorrichtung umfaßt gegebenenfalls eine Multikammer-Transfereinheit zum kontaminationsfreien Transfer von Lösungen aus einer Multikammer-Einheit in eine nachfolgende Multikammer-Einheit und bevorzugt eine Multikammer-Drainageeinheit zum Entfernen von zu verwerfenden Lösungen, die einer Abfall-Sammeleinheit zugeführt werden. Die Drainageeinheit ist bevorzugt mit einer Vakuumvorrichtung gekoppelt. Die Drainageeinheit oder/und Transfereinheit ist bevorzugt verschiebbar angeordnet, wobei in einer operativen Position die Einlaßöffnungen der Drainage-Einheit operativ mit den Auslässen einer Multikammer-Einheit im wesentlichen dichtend verbunden sind, und in einer nicht-operativen Position eine Passage von Flüssigkeiten von der Auslaßseite einer Multikammereinheit und zur Einlaßseite einer nachfolgenden Multikammer-Einheit freigegeben ist. Demgemäß umfaßt die Vorrichtung bevorzugt weiterhin Mittel zum lösbaren Anbringen der Multikammer-Drainageeinheit an der Auslaßseite einer Multikammer-Einheit in der ersten oder/und zweiten Position. Gegebenenfalls kann die Verbindung zwischen Auslaßseite der Multikammer-Einheit und der Multikammer-Drainageeinheit über die Multikammer-Transfereinheit erfolgen. Ebenso können Mittel vorgesehen sein, um eine Multikammer-Transfereinheit in der ersten oder/und zweiten Position zwischen einer operativen und einer nicht-operativen Position zu verschieben.

Weiterhin kann die Vorrichtung Mittel zur Analyse von Zellinhaltsstoffen, wie etwa Elektrophoresevorrichtungen oder/und Spektrometer umfassen, sowie dafür geeignete Steuergeräte oder/und Mittel zur Entnahme von Aliquots aus einer Multikammer-Auffangeinheit oder/und zum Beschicken eines Analysemittels. Weiterhin umfaßtdie Vorrichtung bevorzugt Software, welche die Steuerung mindestens eines Verfahrensschritts erlaubt. Bevorzugt ermöglicht die Software mindestens die Steuerung der Temperatur, der Dauer der Verfahrensschritte sowie der Mittel zur Erzeugung eines Druckunterschieds.

Ein weiterer Gegenstand der Erfindung ist ein Reagenzienkit zur Gewinnung von proteinhaltigen Lösungen aus biologischen Proben im Hochdurchsatzverfahren, umfassend mindestens eine Multikammer-Filtrationseinheit sowie Entschäumer oder/und mindestens eine Multikammertransfer-Einheit. Gegebenenfalls enthält das erfindungsgemäße Reagenzienkit eine oder mehrere weitere Multikammer-Einheiten, beispielweise eine Multikammer-Aufnahmeeinheit.

In einer besonderen Ausführungsform umfaßt das erfindungsgemäße Reagenzienkit weiterhin mindestens ein Reagenz zur Zellyse, bevorzugt zur Zellyse unter Protein-solubilisierenden Bedingungen. Gegebenenfalls umfaßt das Reagenzienkit weiterhin ein oder mehrere Reagenzien zur Solubilisierung von Proteinen oder/und Peptiden.

Das erfindungsgemäße Reagenzienkit umfaßt in einer bevorzugten Ausführungsform Chromatographiematerial. Das Chromatographiematerial ist beispielsweise Material vom Suspensionstyp, das in einem separaten Behälter vorliegt und vom Anwender bei Bedarf in die einzelnen Kammern einer Multikammer-Einheit dispensiert wird. Bevorzugt liegt das Chromatographiematerial bereits in dispensierter Form vor, d.h. in Form einer Multikammer-Chromatographieeinheit. Chromatographiematerialien vom Membrantyp liegen bevorzugt ebenfalls in Form einer Multikammer-Chromatographieeinheit zur unmittelbaren Verwendung im erfindungsgemäßen Verfahren vor.

Das erfindungsgemäße Reagenzienkit enthält darüber hinaus gegebenenfalls herkömmliche Reagenzien wie etwa Äquilibrierungs- und Waschpuffer, oder/und Mittel, welche bei der Durchführung von Verfahrensschritten erforderlich sind, wie beispielsweise Pipettenspitzen.

Die Erfindung wird näher erläutert durch die nachfolgenden Figuren und Beispiele.

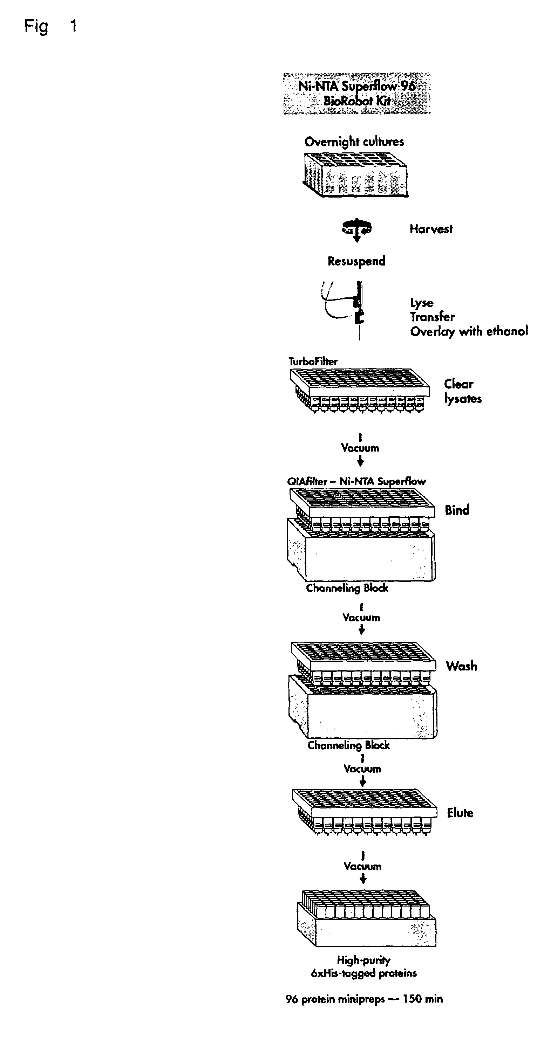

- Figur 1: stellt eine Ausführungsform des erfindungsgemäßen Verfahrens dar;
- Figur 2: zeigt ein SDS-Polyacrylamidgel von gereinigter CAT über Anionenaustauscherchromatographie gemäß Beispiel 3 unter Verwendung einer Matrixsuspension;
- Figur 3a: zeigt ein SDS-Polyacrylamidgel von unterschiedlichen 6xHis-Fusionsproteinen, gereinigt über Metallchelataffinitätschromatographie nach dem erfindungsgemäßen Verfahren gemäß Beispiel 6, und
- Figur 3b: zeigt ein SDS-Polyacrylamidgel von unterschiedlichen 6xHis-Fusionsproteinen, gereinigt über Metallchelataffinitätschromatographie nach einem herkömmlichen Verfahren gem. Beispiel 6 zum Vergleich.

### Beispiele

### 1. Automatisierte Reinigung von 6xHis Fusionsproteinen über Metallaffinitätschromatographie (Matrixsuspension) im 96-Kammer-Format

Dieses Beispiel zeigt die automatisierte Reinigung von Proteinen aus Zellysaten von Escherichia coli (E.coli) über dispensiertes Chromatographiematerial (Ni-NTA Superflow) unter nativen Bedingungen im 96-Kammer-Format unter Anwendung der Vakuumtechnologie.

E.coli DH5*a*-Zellen, die das Protein Chloramphenicol-Acetyl-Transferase als Fusionsprotein mit einer aus sechsmal der Aminosäure Histidin bestehenden Affinitätsmarkierung (6xHis-CAT) exprimieren, wurden in je 5 ml Kulturvolumen in 24-Kammer Blöcken (Fassungsvolumen 10 ml je Kammer; insgesamt 96 Ansätze) gezüchtet und nach Abschluß der Anzucht durch Zentrifugation sedimentiert, überstehendes Kulturmedium wurde entfernt. Alle folgenden Schritte wurden auf einem Laborautomaten (BioRobot 9600, QIAGEN) durchgeführt. Zunächst wurden die Zellsedimente für den Zellaufschluß in je 1 ml Lysepuffer (50 mM NaH₂PO₄, pH 8,0 300 mM NaCl, 20 mM Imidazol, 200 µg/ml Lysozym) aufgenommen und zur Unterstützung eines vollständigen Aufschlusses geschüttelt. Währenddessen wurde Ni-NTA Superflow-Affinitätsmatrix (QIAGEN) resuspendiert und in die Kammern einer weiteren 96-Kammer-Filtrationseinheit dispensiert (50 µl Bettvolumen je Kammer), die Matrix äquilibriert und diese Einheit (Ni-NTA-Platte) in die untere Position einer Vakuumkammer positioniert. In die obere Position exakt über der Ni-NTA-Platte wurde eine Filtrationseinheit (z.B. TurboFilter96, QIAGEN) positioniert. Es erfolgte der Transfer der Lysate auf die TurboFilter 96-Filtrationseinheit; anschließend wurden die Lysate mit je 100 µl Ethanol (100 % p.a.) überschichtet und ein Vakuum angelegt. Anschließend wurde die Turbo-Filter-96-Einheit entfernt, die die klaren Lysate enthaltende Ni-NTA-Platte in die obere Position der Vakuumkammer transferiert und der Drainage-Block in die untere Position gestellt. Durch Anlegen eines Vakuums wurden die klaren Lysate durch das Bett der Chromatographiematrix gesaugt; während dieses Vorgangs erfolgte die Bindung der 6xHis-markierten Proteine an die Matrix. Durch zweimalige Zugabe von 1 ml Puffer (50 mM NaH₂PO₄, pH 8,0; 300 mM NaCI; 20 mM Imidazol) und nachfolgendem Anlegen eines Vakuums wurden die Ansätze gewaschen. Der Drainage-Block in der unteren Position der Vakuumkammer wurde durch eine Mikrotiter-Platte ersetzt. Es erfolgte Zugabe von 300 µl Elutionspuffer (50 mM NaH₂PO₄, pH 8,0, 300 mM NaCI; 250 mM Imidazol) in die einzelnen Kammern und Elution der 6xHis-markierten Proteine in die Kammern der Mikrotiterplatte durch Anlegen eines Vakuums.

Die Proteinkonzentration in den Elutionsfraktionen der Kammern 1 bis 56 wurden durch Bradfordanalyse bestimmt (Tabelle 1). Die Ausbeute an 6xHis-CAT beträgt zwischen 21 und 240 µg pro Kammer (bei einmaliger Elution mit 300 µl). Die gereinigten Proteine sind nach SDS-Polyacrylamidgelelektrophorese von 5 µl jeder Elutionsfraktion und anschließender Coomassie-Brillant-Blue-Färbung der Proteinbanden (SDS-PAGE-Analyse; nicht gezeigt) von apparenter Homogenität.

**Tabelle 1**

| Automatisierte Reinigung von 6xHis-Fusionsprotienen über Metallaffinitätschromatographie (Matrixsuspension) im 96-Kammer-Format | | | | |
|---|---|---|---|---|
| well | Ausbeute [µg] | | well | Ausbeute [µg] |
| A1 | 64 | | C2 | 88 |
| A2 | 108 | | C3 | 84 |
| A3 | 64 | | C4 | 74 |
| A4 | 240 | | C5 | 72 |
| A5 | 67 | | C6 | 91 |
| A6 | 94 | | C7 | 191 |
| A7 | 77 | | C8 | 109 |
| A8 | 65 | | C9 | 70 |
| A9 | 89 | | C10 | 91 |
| A10 | 102 | | C11 | 90 |
| A11 | 61 | | C12 | 60 |
| A12 | 56 | | D1 | 84 |
| B1 | 64 | | D2 | 71 |
| B2 | 111 | | D3 | 88 |
| B3 | 86 | | D4 | 93 |
| B4 | 52 | | D5 | 81 |
| B5 | 90 | | D6 | 21 |
| B6 | 59 | | D7 | 98 |
| B7 | 80 | | D8 | 40 |
| B8 | 111 | | D9 | 77 |
| B9 | 178 | | D10 | 62 |
| B10 | 91 | | D11 | 44 |
| B11 | 104 | | D12 | 135 |
| B12 | 178 | | E1 | 54 |
| C1 | 237 | | E2 | 76 |
| E3 | 71 | | E6 | 91 |
| E4 | 87 | | E7 | 84 |
| E5 | 58 | | E8 | 84 |

| Ausbeute [µg) | |
|---|---|
| Durchschnitt: | 93 |
| Standard-Abweichung | 42 |

### 2. Automatisierte Reinigung von 6xHis Fusionsproteinen über Metallaffinitätschromatographie (Membran) im 96-Kammer-Format

Dieses Beispiel zeigt die automatisierte Reinigung von Proteinen aus Zellysaten von Escherichia coli über eine Affinitätsmembran (einpolymerisierte Ni-NTA Silika-Partikel) unter nativen Bedingungen im 96-Kammer-Format unter Anwendung der Vakuumtechnologie.

Material und Vorgehen für dem Beispiel 2 zugrundeliegenden Experiment entspricht dem für Beispiel 1, außer daß eine 96-Kammer-Platte mit bereits vorher inkorporierter Affinitätsmembran für die Bindung der 6xHis-Fusionsproteine eingesetzt wurde. In die Membran wurden Ni-NTA-Silika-Partikel (QIAGEN) eingearbeitet.

Die Proteinkonzentrationen in den Elutionsfraktionen der Kammern 1 bis 62 wurden durch Bradford-Analyse bestimmt (Tabelle 2). Die Ausbeute an 6xHis-CAT beträgt zwischen 50 und 250 µg pro Kammer (bei einmaliger Elution mit 300 µl). Die gereinigten Proteine sind nach SDS-Polyacrylamidgelelektrophorese von 5 µl jeder Elutionsfraktion und anschließender Coomassie-Brillant-Blue-Färbung der Proteinbanden (SDS-PAGE-Analyse; nicht gezeigt) von apparenter Homogenität.

**Tabelle 2**

| Automatisierte Reinigung von 6xHis-Fusionsproteinen über Metallaffinitätschromatographie (Membran) im 96-Kammer-Format | | | | |
|---|---|---|---|---|
| Well | Ausbeute [µg] | | Well | Ausbeute [µg] |
| A1 | 250 | | C3 | 202 |
| A2 | 169 | | C4 | 151 |
| A3 | 174 | | C5 | 196 |
| A4 | 149 | | C6 | 134 |
| A5 | 179 | | C7 | 145 |
| A6 | 93 | | C8 | 128 |
| A7 | 152 | | C9 | 147 |
| A8 | 131 | | C10 | 184 |
| A9 | 171 | | C11 | 137 |
| A10 | 156 | | C12 | 103 |
| A11 | 123 | | D1 | 91 |
| A12 | 106 | | D2 | 117 |
| B1 | 225 | | D3 | 168 |
| B2 | 176 | | D4 | 90 |
| B3 | 148 | | D5 | 73 |
| B4 | 153 | | D6 | 131 |
| B5 | 148 | | D7 | 137 |
| B6 | 143 | | D8 | 117 |
| B7 | 129 | | D9 | 160 |
| B8 | 137 | | D10 | 113 |
| B9 | 126 | | D11 | 145 |
| B10 | 130 | | D12 | 93 |
| B11 | 246 | | E1 | 158 |
| B12 | 102 | | E2 | 127 |
| C1 | 129 | | E3 | 166 |
| C2 | 190 | | E4 | 49 |
| E5 | 121 | | E10 | 147 |
| E6 | 59 | | E11 | 130 |
| E7 | 170 | | E12 | 129 |
| E8 | 98 | | F1 | 172 |
| E9 | 145 | | F2 | 131 |

| Ausbeute [µg) | |
|---|---|
| Durchschnitt | 142 |
| Standard-Abweichung | 39 |

### 3. Automatisierte Reinigung von Chloramphenicol-Acetyl-Transferase (CAT) über Anionentauscherchromatographie (Matrixsuspension) im 96-Kammer-Format

Dieses Beispiel zeigt die automatisierte Reinigung des Proteins CAT aus Zellysaten von Escherichia coli über dispensiertes Anionentauscher-Chromatographiematerial (Q-Sepharose Fast Flow) unter nativen Bedingungen im 96-Kammer-Format unter Anwendung der Vakuumtechnologie.

Material und Vorgehen für dem Beispiel 3 zugrundeliegenden Experiment entspricht dem für Beispiel 1 mit einer Änderung. Dabei wurde die Tatsache genutzt, daß Chloramphenicol-Acetyl-Transferase (CAT) über Anionentauscherchromatographie gereinigt werden kann. Im hier beschriebenen Versuch wurde eine 96-Kammer-Platte mit dispensierter Suspension der Q-Sepharose Fast Flow-Anionentauschermatrix (Amersham Pharmacia Biotech) für die Bindung des rekombinanten Proteins eingesetzt (Lysepuffer: 50 mM Tris HCl, 25 mM NaCI, 200 µg/ml Lysozym, pH 7,0; Bindungspuffer/Waschpuffer: 50 mM Tris HCl, 25 mM NaCI, pH 7,0; Elutionspuffer: 50 mM Tris HCI, 500 mM NaCI, pH 7,0).

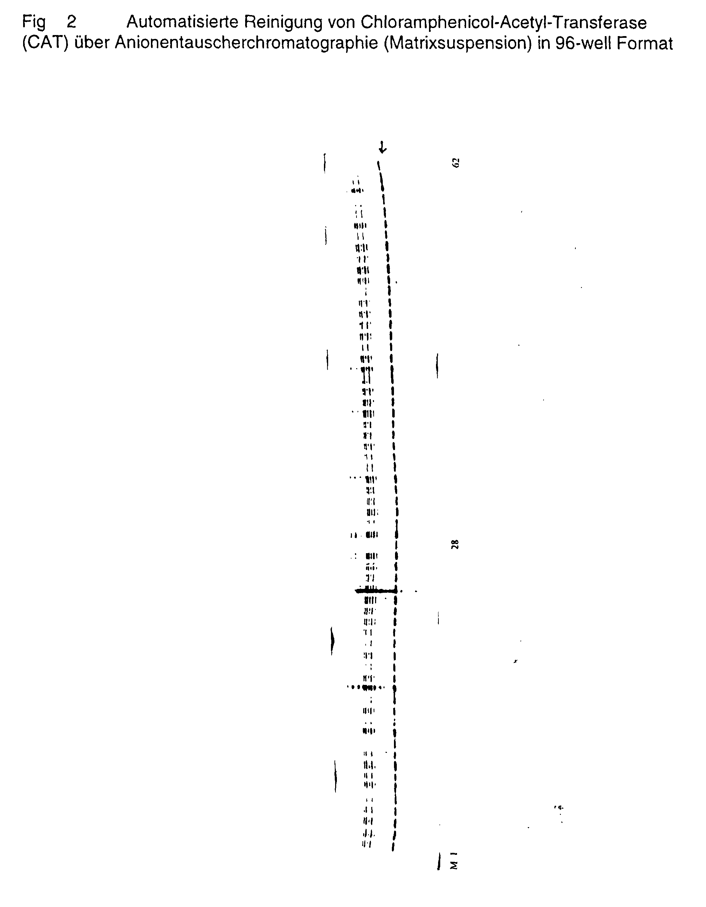

Die Proteinkonzentrationen in den Elutionsfraktionen der Kammern 1 bis 62 wurden durch Bradford-Analyse bestimmt (Tabelle 3). 5 µl jeder Fraktion wurden auf ein SDS-Polyacrylamidgel (SDS-PAGE, 15%) geladen. Die Proteinbanden im Gel wurden mittels Coomassie-Brillant-Blue-Färbung sichtbar gemacht (Figur 2). CAT konnte danach zu einer Reinheit von ca. 40% angereichert werden. Die Ausbeute von CAT beträgt danach zwischen 40 und 120 µg pro Kammer (bei einmaliger Elution mit 200 µl).

**Tabelle 3**

| Automatisierte Reinigung von Chloramphenicol-Acetyl-Transferase über Anionentauscherchromatographie (Matrixsuspension) im 96-Kammer-Format | | | | |
|---|---|---|---|---|
| Well | Ausbeute [µg] | | Well | Ausbeute [mg] |
| A1 | 152 | | C3 | 152 |
| A2 | 125 | | C4 | 266 |
| A3 | 116 | | C5 | 157 |
| A4 | 151 | | C6 | 222 |
| A5 | 206 | | C7 | 138 |
| A6 | 127 | | C8 | 163 |
| A7 | 213 | | C9 | 125 |
| A8 | 194 | | C10 | 166 |
| A9 | 215 | | C11 | 159 |
| A10 | 121 | | C12 | 108 |
| A11 | 107 | | D1 | 149 |
| A12 | 162 | | D2 | 146 |
| B1 | 156 | | D3 | 141 |
| B2 | 110 | | D4 | 132 |
| B3 | 134 | | D5 | 117 |
| B4 | 162 | | D6 | 240 |
| B5 | 213 | | D7 | 192 |
| B6 | 209 | | D8 | 209 |
| B7 | 161 | | D9 | 147 |
| B8 | 133 | | D10 | 206 |
| B9 | 135 | | D11 | 153 |
| B10 | 176 | | D12 | 112 |
| B11 | 147 | | E1 | 118 |
| B12 | 108 | | E2 | 169 |
| C1 | 98 | | E3 | 165 |
| C2 | 158 | | E4 | 301 |
| E5 | 158 | | E10 | 149 |
| E6 | 228 | | E11 | 204 |
| E7 | 301 | | E12 | 110 |
| E8 | 191 | | F1 | 172 |
| E9 | 226 | | F2 | 131 |

| Ausbeute [µg) | |
|---|---|
| Durchschnitt | 165 |
| Standard-Abweichung | 46 |

### 4. Automatisierte Reinigung von eukaryontisch exprimierten 6xHis Fusionsproteinen über Metallaffinitätschromatographie (Matrixsuspension) im 96-Kammer-Format

Dieses Beispiel beschreibt die Reinigung von 6xHis Fusionsproteinen aus Zellysaten der Hefe Saccharomyces cerevisiae über dispensiertes Chromatographiematerial (Ni-NTA Superflow) unter nativen Bedingungen im 96-Kammer-Format unter Anwendung der Vakuumtechnologie.

Material und Vorgehen für dem Beispiel 4 zugrundeliegenden Experiment entspricht dem für Beispiel 1 mit der Änderung, daß im hier beschriebenen Versuch das in eukaryotischen Saccharomyces cerevisiae-Zellen exprimierte, 6xHis-markierte Protein FBA1-Aldolase gereinigt wurde. Dazu wurden die Lysebedingungen wie folgt verändert: Die in 24-Kammer Blöcken in je 5 ml Kulturmedium angezogenen Zellen wurden in Lysepuffer (20 mM KH₂PO₄/K₂HPO₄, pH 7,4; 1,2 M Sorbitol; 1 mg/ml Zymolyase) resuspendiert und zur Unterstützung der Sphäroplastenbildung 45 Minuten geschüttelt. Im Anschluß wurde Protease-Inhibitor, 300 mM NaCI sowie 0,4 % (v/v) Triton X-100 zur Sphäroplastenlyse zugegeben und die Lyseansätze zur Klärung auf eine Filtrationseinheit (z.B. Turbo-Filter 96, QIAGEN) transferiert. Alle folgenden Schritte wurden wie für Beispiel 1 beschrieben durchgeführt.

Die Proteinkonzentrationen in den Elutionsfraktionen wurden durch Bradford-Analyse bestimmt (Tabelle 4). Die Ausbeute an 6xHis-FBA1-Aldolase betrug zwischen 10 und 25 µg pro Kammer. Die gereinigten Proteine sind nach SDS-PAGE-Analyse (nicht gezeigt) von apparenter Homogenität.

**Tabelle 4**

| Automatisierte Reinigung von eukaryontisch exprimierten 6xHis-Fusionsproteinen über Metallaffinitätschromatographie (Matrixsuspension) im 96-Kammer-Format | | | | |
|---|---|---|---|---|
| Kammer | Ausbeute [µg] | | Kammer | Ausbeute [µg] |
| A1 | 15 | | E1 | 16 |
| A2 | 16 | | E2 | 16 |
| A3 | 11 | | E3 | 12 |
| A4 | 21 | | E4 | 15 |
| A5 | 13 | | E5 | 17 |
| A6 | 19 | | E5 | 17 |
| B1 | 16 | | F1 | 15 |
| B2 | 24 | | F2 | 16 |
| B3 | 8 | | F3 | 22 |
| B4 | 9 | | F4 | 10 |
| B5 | 17 | | F5 | 19 |
| B6 | 15 | | F6 | 14 |
| C1 | 16 | | G1 | 12 |
| C2 | 10 | | D2 | 12 |
| C3 | 16 | | G2 | 8 |
| C4 | 12 | | D3 | 21 |
| C5 | 11 | | G3 | 15 |
| C6 | 17 | | D4 | 18 |
| D1 | 18 | | H1 | 11 |
| D2 | 14 | | H2 | 16 |
| D3 | 17 | | H3 | 10 |
| D4 | 16 | | H4 | 16 |
| D5 | 18 | | H5 | 13 |
| D6 | 15 | | H6 | 19 |

| Ausbeute [µg] | |
|---|---|
| Durchschnitt | 15 |
| Standard-Abweichung | 4 |

### 5. Automatisierte Reinigung von 6xHis markierten Fusionsproteinen über Metallaffinitätschromatographie (Matrixsuspension) im 96-Kammer-Format unter denaturierenden Bedingungen

Dieses Beispiel zeigt die automatisierte Reinigung von Proteinen aus Zellysaten von Escherichia coli über dispensierte Chromatographiematerial (Ni-NTA Superflow) unter denaturierenden Bedingungen im 96-Kammer-Format unter Anwendung der Vakuumtechnologie.

E.coli M15/pREP4-Zellen, die Thioredoxin als Fusionsprotein mit einer aus sechsmal der Aminosäure Histidin bestehenden Affinitätsmarkierung (6xHis) exprimieren, wurden in je 5 ml Kulturvolumen in 24-Kammer-Blöcken (Fassungsvolumen 10 ml je Kammer; insgesamt 96 Ansätze) gezüchtet und nach Abschluß der Anzucht durch Zentrifugation sedimentiert; überstehendes Kulturmedium wurde entfernt. Alle folgenden Schritte wurden auf einem Laborautomaten (BioRobot 9600, QIAGEN) durchgeführt. Zunächst wurden die Zellsedimente für den Zellaufschluß in je 1 ml Lysepuffer (8 M Harnstoff, 100 mM NaH₂PO₄, 10 mM Tris HCI, pH 8,0) aufgenommen und zur Unterstützung eines vollständigen Aufschlusses 30 min geschüttelt. Währenddessen wurde Ni-NTA Superflow-Affinitätsmatrix resuspendiert und in die Kammern einer weiteren 96-Kammer-Einheit dispensiert (50 µl Bettvolumen je Kammer), die Matrix äquilibriert und diese Einheit (Ni-NTA-Platte) in die untere Position einer Vakuumkammer positioniert. In die obere Position exakt über der Ni-NTA-Platte wurde eine Filtrationseinheit (z.B. TurboFilter 96, QIAGEN) positioniert. Es erfolgte der Transfer der Lysate auf die TurboFilter 96-Filtrationseinheit; anschließend wurden die Lysate mit je 100 µl Ethanol (p.a.) überschichtet und ein Vakuum angelegt. Nach vollständiger Filtration wurde die TurboFilter 96-Einheit entfernt, die die klaren Lysate enthaltende Ni-NTA-Platte in die obere Position der Vakuumkammer transferiert und der Drainage-Block in die untere Position gestellt. Durch Anlegen eines Vakuums wurden die klaren Lysate durch das Bett der Chromatographiematrix gesaugt; während dieses Vorgangs erfolgte die Bindung der 6xHis-markierten Proteine an die Matrix. Durch zweimalige Zugabe von 1 ml Waschpuffer 1 (8 M Harnstoff, 100 mM NaH₂PO₄, 10 mM Tris HCI, pH 8,0) und einmalige Zugabe von Waschpuffer 2 (8 M Harnstoff, 100 mM NaH₂PO₄, 10 mM Tris HCl, pH 6,3) und nachfolgendem Anlegen eines Vakuums wurden die Ansätze gewaschen. Der Drainage-Block in der unteren Position der Vakuumkammer wurde durch eine Mikrotiter-Platte ersetzt. Es erfolgte Zugabe von je 300 µl Elutionspuffer (8 M Harnstoff, 100 mM NaH₂PO₄, 10 mM Tris HCI, pH 4,5) in die einzelnen Kammern und Elution der 6xHis-markierten Proteine in die Kammern der Mikrotiterplatte durch Anlegen eines Vakuums.

Die Proteinkonzentrationen in den Elutionsfraktionen der Kammern 1 bis 54 wurden durch Bradford-Analyse bestimmt (Tabelle 5). Die Ausbeute an 6xHis-markiertem Protein beträgt zwischen 86 und 252 µg pro well. Die gereinigten Proteine sind nach SDS-PAGE-ANALYSE (nicht gezeigt) von apparenter Homogenität.

**Tabelle 5**

| well | Ausbeute [µg] | | well | Ausbeute [µg] |
|---|---|---|---|---|
| A1 | 110 | | C2 | 118 |
| A2 | 131 | | C3 | 147 |
| A3 | 185 | | C4 | 132 |
| A4 | 198 | | C5 | 147 |
| A5 | 100 | | C6 | 118 |
| A6 | 114 | | C7 | 155 |
| A7 | 192 | | C8 | 180 |
| A8 | 156 | | C9 | 171 |
| A9 | 248 | | C10 | 186 |
| A10 | 180 | | C11 | 152 |
| A11 | 161 | | C12 | 145 |
| A12 | 160 | | D1 | 112 |
| B1 | 129 | | D2 | 174 |
| B2 | 130 | | D3 | 152 |
| B3 | 143 | | D4 | 156 |
| B4 | 197 | | D5 | 111 |
| B5 | 142 | | D6 | 97 |
| B6 | 221 | | D7 | 216 |
| B7 | 203 | | D8 | 220 |
| B8 | 157 | | D9 | 171 |
| B9 | 182 | | D10 | 155 |
| B10 | 153 | | D11 | 151 |
| B11 | 167 | | D12 | 141 |
| B12 | 156 | | E1 | 104 |
| C1 | 131 | | E2 | 153 |
| E3 | 140 | | E8 | 174 |
| E4 | 137 | | E9 | 115 |
| E5 | 86 | | E10 | 196 |
| E6 | 183 | | E11 | 196 |
| E7 | 252 | | E12 | 143 |
| | | | F1 | 150 |
| | | | F2 | 159 |

| Ausbeute [µg] | |
|---|---|
| Durchschnitt | 156 |
| Standard-Abweichung | 36 |

### 6. Kreuzkontaminationsfreie Reinigung von unterschiedlichen 6xHis Fusionsproteinen über Metallaffinitätschromatographie im 96-Kammer Format

Dieses Beispiel beschreibt die automatisierte Reinigung von Proteinen aus Zellysaten von Escherichia coli über dispensiertes Chromatographiematerial (Ni-NTA Superflow) unter denaturierenden Bedingungen im 96-Kammer-Format unter Anwendung der Vakuumtechnologie unter Einbeziehung von Maßnahmen zur Vermeidung von Kreuzkontaminationen im Vergleich zu einer Reinigung, die ohne die beschriebenen Maßnahmen durchgeführt wurde.

Material und Vorgehen für dem Beispiel 6 zugrundeliegenden Experiment entspricht dem für Beispiel 5, außer daß unterschiedliche 6xHis Fusionsproteine exprimierende Klone in je 5 ml Medium kultiviert und parallel aufgearbeitet wurden. Die Maßnahmen, die zur Vermeidung von Kreuzkontaminationen getroffen wurden (Überschichten mit Ethanol, Verwendung eines Drainage-Blocks) sind im Beispiel 4 beschrieben.

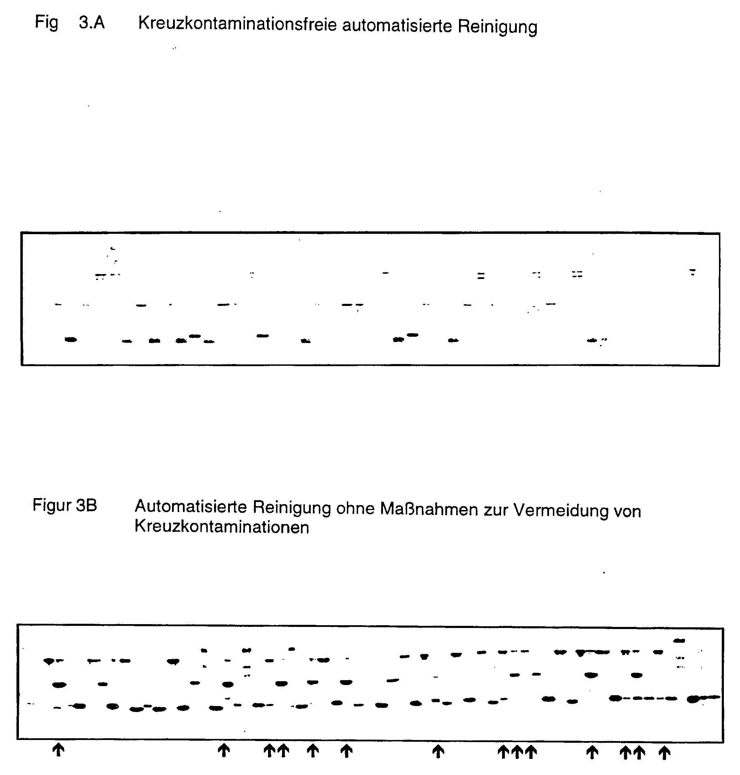

Je 5 µl der angegebenen Elutionsfraktionen wurden für 4 min bei 95°C erhitzt und durch SDS-PAGE und anschließende Coomassie-Färbung der Proteinbanden analysiert (Abbildung 3.). Das Bandenmuster der Elutionsfraktionen bei Reinigung unter Verwendung des erfindungsgemäßen Verfahrens (Abbildung 3a) zeigt, daß in den Fraktionen jeweils nur eine einem der 6xHis Fusionsproteine entsprechende Bande auftritt, Kreuzkontaminationen durch diese Methode somit ausgeschlossen werden können. Demgegenüber können in dem Kontrollexperiment zum Teil mehrere einem 6xHis Fusionsprotein entsprechende Banden identifiziert werden (Abbildung 3b, mit ↑ gekennzeichneten Bahnen).

## Patentansprüche

1. Verfahren zur Gewinnung von Zellinhaltsstoffe enthaltenden klaren Lösungen aus biologischen Proben im Hochdurchsatzverfahren, umfassend die Schritte:
(a) Bereitstellen von mehreren proteinhaltigen Lösungen, die unlösliche Bestandteile enthalten, in separaten Kammern einer Multikammer-Filtrationseinheit,
(b) Entfernen von unlöslichen Bestandteilen durch Filtrieren der Lösungen durch die Multikammer-Filtrationseinheit unter Anlegen einer Druckdifferenz, wobei durch Zugabe eines Entschäumers zu den Zelllysaten oder/und durch Verwendung einer Transfereinheit zwischen Auslassseite der Multikammer-Filtrationseinheit und Einlassseite der Auffangbehälter eine Kreuzkontamination benachbarter Kammern verhindert wird und
(c) Auffangen der einzelnen Filtrate jeweils separat in Auffangbehältern.

2. Verfahren nach Anspruch 1,
**dadurch gekennzeichnet,**
**dass** der Entschäumer ausgewählt wird aus primären, sekundären oder tertiären C1-C6-Alkoholen, silikonischen Entschäumern, nichtsilikonischen Entschäumern, Entschäumern auf vegetabilischer Basis, langkettigen Alkanen, Polypropylenglykolen, Polyethylenglykolen und Gemischen davon.

3. Verfahren nach Anspruch 1 oder 2,
**dadurch gekennzeichnet,**
**dass** die proteinhaltigen Lösungen nach (a) Zell-Rohlysate sind.

4. Verfahren nach Anspruch 3,
**dadurch gekennzeichnet,**
**dass** man die Zelllyse jeweils in den separaten Kammern der Multikammer-Filtrationseinheit durchführt.

5. Verfahren nach einem der Ansprüche 1 bis 4,
**dadurch gekennzeichnet,**
**dass** die Porengröße der Filter in der Multikammer-Filtrationseinheit in Fließrichtung des Filtrats durch den Filter abnimmt.

6. Verfahren nach einem der Ansprüche 1 bis 5, weiterhin umfassend das Gewinnen von Zellinhaltsstoffen aus Filtraten nach (c).

7. Verfahren nach Anspruch 6, umfassend mindestens einen weiteren Filtrationsschritt.

8. Verfahren nach Anspruch 6 oder 7, umfassend mindestens einen chromatografischen Trennschritt oder/und Präzipitationsschritt.

9. Verfahren nach Anspruch 8, umfassend einen chromatografischen Trennschritt über eine lonenaustauschmatrix oder eine Affinitätsmatrix.

10. Verfahren nach Anspruch 9,
**dadurch gekennzeichnet,**
**dass** die Affinitätsmatrix eine Metallchelat-Affinitätsmatrix ist.

11. Verfahren nach Anspruch 10,
**dadurch gekennzeichnet,**
**dass** die Metallchelat-Affinitätsmatrix eine Ni-NTA-Matrix ist.

12. Verfahren nach einem der Ansprüche 9 bis 11,
**dadurch gekennzeichnet,**
**dass** die Matrix eine Suspension ist.

13. Verfahren nach einem der Ansprüche 6 bis 12, weiterhin umfassend mindestens einen Waschschritt.

14. Verfahren nach einem der Ansprüche 6 bis 13,
**dadurch gekennzeichnet,**
**dass** man das Abtrennen von Flüssigkeiten durch Anlegen einer Druckdifferenz durchführt.

15. Verfahren nach Anspruch 14,
**dadurch gekennzeichnet,**
**dass** man zu verwerfende Flüssigkeiten unter Verwendung einer Drainageeinheit entfernt.

16. Verfahren nach einem der Ansprüche 6 bis 15, weiterhin umfassend mindestens einen Schritt zur Analyse der Zellinhaltsstoffe.

17. Verfahren nach einem der Ansprüche 6 bis 16,
**dadurch gekennzeichnet,**
**dass** man die Zellinhaltsstoffe in einer Multikammer-Einheit auffängt.

18. Verfahren nach einem der Ansprüche 6 bis 17,
**dadurch gekennzeichnet,**
**dass** die Zellinhaltsstoffe ausgewählt werden aus Peptiden, Polypeptiden, Nukleinsäuren und Metaboliten.

19. Verwendung einer Vorrichtung, umfassend:
(i) Mittel zur Halterung einer Multikammer-Einheit in einer ersten Position,
(ii) Mittel zur Halterung einer Multikammer-Einheit in einer zweiten Position,
(iii) Mittel zum Anlegen einer Druckdifferenz zwischen der Einlassseite und der Auslassseite mindestens der Multikammer-Einheit in der ersten Position und
(iv) Mittel zur Zugabe von Reagenzien zu einzelnen Kammern mindestens der Multikammer-Einheit in der ersten Position
zur Gewinnung von klaren proteinhaltigen Lösungen aus biologischen Proben.

20. Verwendung nach Anspruch 19,
**dadurch gekennzeichnet,**
**dass** die Multikammer-Einheiten derart ausgerichtet sind, dass die Auslässe der Kammern einer Einheit in der ersten Position mit den Einlässen der Kammern einer Einheit in der zweiten Position fluchten.

21. Verwendung nach Anspruch 19 oder 20,
**dadurch gekennzeichnet,**
**dass** die Vorrichtung:
(i) Mittel zum Entfernen einer Multikammer-Einheit aus der ersten Position,
(ii) Mittel zum Transferieren einer Multikammer-Einheit von der zweiten in die erste Position und
(iii) Mittel zum Transferieren einer Multikammer-Einheit in die zweite Position
umfasst.

22. Verwendung nach einem der Ansprüche 19 bis 21,
**dadurch gekennzeichnet,**
**dass** die Vorrichtung weiterhin eine Multikammer-Transfereinheit umfasst.

23. Verwendung nach einem der Ansprüche 19 bis 22,
**dadurch gekennzeichnet,**
**dass** die Vorrichtung weiterhin eine Multikammer-Drainageeinheit umfasst.

24. Verwendung nach Anspruch 23,
**dadurch gekennzeichnet,**
**dass** die Vorrichtung weiterhin Mittel zum lösbaren Anbringen der Multikammer-Drainageeinheit an der Auslassseite einer Multikammer-Einheit in der ersten oder/und zweiten Position umfasst.

25. Verwendung nach einem der Ansprüche 19 bis 24,
**dadurch gekennzeichnet,**
**dass** die Vorrichtung weiterhin Mittel zur Analyse von Zellinhaltsstoffen umfasst.

26. Verwendung nach einem der Ansprüche 19 bis 25,
**dadurch gekennzeichnet,**
**dass** die Vorrichtung weiterhin Software zur Steuerung mindestens eines Verfahrensschrittes umfasst.

27. Reagenzienkit zur Gewinnung von proteinhaltigen Lösungen aus biologischen Proben im Hochdurchsatzverfahren, umfassend:
(a) mindestens eine Multikammer-Filtrationseinheit,
(b) Entschäumer oder/und mindestens eine Multikammer-Transfereinheit und
(c) gegebenenfalls mindestens eine Multikammer-Aufnahmeeinheit.

28. Reagenzienkit nach Anspruch 27, weiterhin umfassend mindestens ein Reagenz zur Zelllyse.

29. Reagenzienkit nach Anspruch 27 oder 28, weiterhin umfassend mindestens ein Reagenz zur Solubilisierung von Proteinen oder/und Peptiden.

30. Reagenzienkit nach einem der Ansprüche 27 bis 29, weiterhin umfassend Chromatografiematerial.

31. Reagenzienkit nach Anspruch 30, wobei das Chromatografiematerial in Form mindestens einer Muitikammer-Chromatografieeinheit vorliegt.

## Claims

1. Process for recovering clear solutions containing cell contents from biological samples in a high throughput process, comprising the steps of
(a) preparing a plurality of protein-containing solutions which contain insoluble ingredients in separate chambers of a multi-chamber filtration unit,
(b) removing insoluble ingredients by filtering the solutions through the multi-chamber filtration unit with the application of a pressure differential, cross contamination of adjacent chambers being prevented by the addition of an antifoam to the cell lysates and/or by the use of a transfer unit between the outlet end of the multi-chamber filtration unit and the inlet end of the collecting containers, and
(c) collecting the individual filtrates separately in collecting containers.

2. Process according to claim 1, **characterised in that** the antifoam is selected from primary, secondary or tertiary C₁₋₆ alcohols, silicone antifoams, non-silicone antifoams, plant-based antifoams, long-chained alkanes, polypropylene glycols, polyethylene glycols and mixtures thereof.

3. Process according to claim 1 or 2, **characterised in that** the solutions containing protein according to (a) are crude cell lysates.

4. Process according to claim 3, **characterised in that** the cell lysis is carried out in the separate chambers of the multi-chamber filtration unit.

5. Process according to one of claims 1 to 4, **characterised in that** the pore size of the filters in the multi-chamber filtration unit decreases in the direction of flow of the filtrate through the filter.

6. Process according to one of claims 1 to 5, further comprising recovering cell contents from filtrates according to (c).

7. Process according to claim 6, comprising at least one further filtration step.

8. Process according to claim 6 or 7, comprising at least one chromatographic separation step and/or precipitation step.

9. Process according to claim 8, comprising a chromatographic separation step using an ion exchange matrix or an affinity matrix,

10. Process according to claim 9, **characterised in that** the affinity matrix is a metal chelate affinity matrix.

11. Process according to claim 10, **characterised in that** the metal chelate affinity matrix is an Ni-NTA matrix.

12. Process according to one of claims 9 to 11, **characterised in that** the matrix is a suspension.

13. Process according to one of claims 6 to 12, further comprising at least one washing step.

14. Process according to one of claims 6 to 13, **characterised in that** the liquids are separated by the application of a pressure differential.

15. Process according to claim 14, **characterised in that** liquids to be discarded are eliminated using a drainage unit.

16. Process according to one of claims 6 to 15, further comprising at least one step of analysing the cell contents.

17. Process according to one of claims 6 to 16, **characterised in that** the cell contents are collected in a multi-chamber unit.

18. Process according to one of claims 6 to 17, **characterised in that** the cell contents are selected from peptides, polypeptides, nucleic acids and metabolites.

19. Use of an apparatus, comprising:
(i) means for holding a multi-chamber unit in a first position,
(ii) means for holding a multi-chamber unit in a second position,
(iii) means for applying a pressure differential between the inlet end and the outlet end of at least the multi-chamber unit in the first position and
(iv) means for adding reagents to individual chambers of at least the multi-chamber unit in the first position,
in order to recover clear protein-containing solutions from biological samples.

20. Use according to claim 19, **characterised in that** the multi-chamber units are aligned so that the outlets of the chambers of a unit in the first position are in alignment with the inlets of the chambers of a unit in the second position.

21. Use according to claim 19 or 20, **characterised in that** the apparatus comprises
(i) means for removing a multi-chamber unit from the first position,
(ii) means for transferring a multi-chamber unit from the second to the first position, and
(iii) means for transferring a multi-chamber unit to the second position.

22. Use according to one of claims 19 to 21, **characterised in that** the apparatus further comprises a multi-chamber transfer unit.

23. Use according to one of claims 19 to 22, **characterised in that** the apparatus further comprises a multi-chamber drainage unit.

24. Use according to claim 23, **characterised in that** the apparatus further comprises means for releasably attaching the multi-chamber drainage unit to the outlet end of a multi-chamber unit in the first and/or second position.

25. Use according to one of claims 19 to 24, **characterised in that** the apparatus further comprises means for analysing cell contents.

26. Use according to one of claims 19 to 25, **characterised in that** the apparatus further comprises software for controlling at least one process step.

27. Reagent kit for obtaining protein-containing solutions from biological samples in a high throughput process, comprising
(a) at least one multi-chamber filtration unit,
(b) antifoams and/or at least one multi-chamber transfer unit and
(c) optionally at least one multi-chamber receiving unit.

28. Reagent kit according to claim 27, further comprising at least one reagent for cell lysis.

29. Reagent kit according to claim 27 or 28, further comprising at least one reagent for solubilising proteins and/or peptides.

30. Reagent kit according to one of claims 27 to 29, further comprising chromatographic material.

31. Reagent kit according to claim 30, wherein the chromatographic material is in the form of at least one multi-chamber chromatography unit.

## Revendications

1. Procédé pour l'obtention de solutions limpides contenant des matières cellulaires à partir d'échantillons biologiques dans un procédé à haut débit, comprenant les étapes de :
a) préparer plusieurs solutions contenant des protéines, qui contiennent des composants insolubles, dans les chambres séparées d'une unité de filtration à chambres multiples,
b) retirer les composants insolubles par filtration des solutions par l'unité de filtration à chambres multiples en appliquant une pression différentielle, de sorte qu'en ajoutant un agent antimoussant aux lysats de cellules ou/et en utilisant une unité de transfert entre le côté sortie de l'unité de filtration à chambres multiples et le côté entrée des récipients de réception on empêche une contamination croisée de chambres avoisinantes et
c) réceptionner les filtrats individuels chacun séparément dans des récipients de réception.

2. Procédé selon la revendication 1,
**caractérisé en ce que**,
l'agent antimoussant est sélectionné à partir d'alcools en C1-C6 primaires, secondaires ou tertiaires, d'agents antimoussants à base de silicone, d'agents antimoussants qui ne sont pas à base de silicone, d'agents antimoussants à base végétale, d'alcanes à chaînes longues, de polypropylènes-glycols, de polyéthylènes-glycols et de leurs mélanges.

3. Procédé selon la revendication 1 ou 2,
**caractérisé en ce que**,
les solutions contenant des protéines sont des lysats bruts de cellules selon a).

4. Procédé selon la revendication 3,
**caractérisé en ce que**,
l'on conduit la lyse cellulaire respectivement dans les chambres séparées de l'unité de filtration à chambres multiples.

5. Procédé selon l'une des revendications 1 à 4,
**caractérisé en ce que**,
la taille des pores des filtres dans l'unité de filtration à chambres multiples va en s'amenuisant dans la direction d'écoulement du filtrat à travers le filtre.

6. Procédé selon l'une des revendications 1 à 5, comprenant de plus l'obtention de matières cellulaires à partir des filtrats selon c).

7. Procédé selon la revendication 6, comprenant au moins une autre étape de filtration.

8. Procédé selon la revendication 6 ou 7, comprenant au moins une étape de séparation chromatographique ou/et une étape de précipitation.

9. Procédé selon la revendication 8, comprenant une étape de séparation chromatographique par le biais d'une matrice échangeuse d'ions ou d'une matrice d'affinité.

10. Procédé selon la revendication 9,
**caractérisé en ce que**,
la matrice d'affinité est une matrice d'affinité au chélate métallique.

11. Procédé selon la revendication 10,
**caractérisé en ce que**,
la matrice d'affinité au chélate métallique est une matrice Ni-NTA.

12. Procédé selon l'une des revendications 9 à 11,
**caractérisé en ce que**,
la matrice est une suspension.

13. procédé selon l'une des revendications 6 à 12, comprenant de plus au moins une étape de lavage.

14. Procédé selon l'une des revendications 6 à 13,
**caractérisé en ce que**,
l'on effectue la séparation des liquides par application d'une pression différentielle.

15. Procédé selon la revendication 14,
**caractérisé en ce que**,
l'on élimine les liquides à rejeter en utilisant une unité de drainage.

16. Procédé selon l'une des revendications 6 à 15, comprenant de plus au moins une étape pour l'analyse des matières cellulaires.

17. Procédé selon l'une des revendications 6 à 16,
**caractérisé en ce que**,
l'on réceptionne les matières cellulaires dans une unité à chambres multiples.

18. Procédé selon l'une des revendications 6 à 17,
**caractérisé en ce que**,
les matières cellulaires sont choisies à partir de peptides, polypeptides, acides nucléiques et métabolites.

19. Utilisation d'un dispositif comprenant :
(i) des moyens pour fixer une unité à chambres multiples dans une première position,
(ii) des moyens pour fixer une unité à chambres multiples dans une deuxième position,
(iii) des moyens pour appliquer une pression différentielle entre le côté entrée et le côté sortie au moins de l'unité à chambres multiples dans la première position et
(iv) des moyens pour ajouter des réactifs aux différentes chambres au moins de l'unité à chambres multiples dans la première position
pour l'obtention de solutions limpides contenant des protéines à partir d'échantillons biologiques.

20. utilisation selon la revendication 19,
**caractérisée en ce que**,
les unités à chambres multiples sont orientées de telle sorte que les sorties des chambres dans la première position sont alignées sur les entrées des chambres d'une unité dans la deuxième position.

21. Utilisation selon la revendication 19 ou 20,
**caractérisée en ce que**,
le dispositif comprend :
(i) des moyens pour retirer une unité à chambres multiples de la première position,
(ii) des moyens pour transférer une unité à chambres multiples de la deuxième à la première position et
(iii) des moyens pour transférer une unité à chambres multiples dans la deuxième position.

22. Utilisation selon l'une des revendications 19 à 21,
**caractérisée en ce que**,
le dispositif comprend de plus une unité de transfert à chambres multiples.

23. Utilisation selon l'une des revendications 19 à 22,
**caractérisée en ce que**,
le dispositif comprend de plus une unité de drainage à chambres multiples.

24. Utilisation selon la revendication 23,
**caractérisée en ce que**,
le dispositif comprend de plus des moyens pour la mise en place amovible de l'unité de drainage à chambres multiples sur le côté sortie d'une unité à chambres multiples dans la première ou/et dans la deuxième position.

25. Utilisation selon l'une des revendications 19 à 24,
**caractérisée en ce que**,
le dispositif comprend de plus des moyens pour l'analyse des matières cellulaires.

26. Utilisation selon l'une des revendications 19 à 25,
**caractérisée en ce que**,
le dispositif comprend de plus un logiciel pour la commande d'au moins une étape du procédé.

27. Kit de réactifs pour l'obtention de solutions contenant des protéines à partir d'échantillons biologiques dans un procédé à haut débit, comprenant :
a) au moins une unité de filtration à chambres multiples,
b) des agents antimoussants ou/et au moins une unité de transfert à chambres multiples et
c) éventuellement au moins une unité de réception à chambres multiples.

28. Kit de réactifs selon la revendication 27, comprenant de plus au moins un réactif pour la lyse cellulaire.

29. Kit de réactifs selon la revendication 27 ou 28, comprenant de plus au moins un réactif pour la solubilisation de protéines ou/et de peptides.

30. Kit de réactifs selon l'une des revendications 27 à 29, comprenant de plus un matériau de chromatographie.

31. Kit de réactifs selon la revendication 30, dans lequel le matériau de chromatographie se présente sous la forme d'au moins une unité de chromatographie à chambres multiples.
